# EUROPEAN PATENT APPLICATION

(11) **EP 4 697 747 A1**
(43) Date of publication of application: **18.02.2026**
(21) Application number: 25195643.9
(22) Date of filing: 13.08.2025
(51) Int. Cl.: H04R 1/10, A61B 5/12, H04R 25/00

(54) **TRANSDUCER FIXTURE FOR AN EAR PROBE**

(30) Priority: 14.08.2024 EP 24194547
(71) Applicant: Interacoustics A/S, 5500 Middelfart (DK)
(72) Inventor: SJURSEN, Jonas, DK-5500 Middelfart (DK); NØRGAARD, Kren Monrad, DK-5500 Middelfart (DK)
(74) Representative: Demant

(57) **Abstract**

A transducer fixture for an ear probe, the use of a transducer fixture in an ear probe of an instrument for hearing diagnostics, an ear probe for hearing diagnostics comprising a transducer fixture and a method for producing a transducer fixture for an ear probe are disclosed. The transducer fixture for an ear probe comprises: an interface for a probe tip, at least one opening for attaching a transducer, at least one channel for acoustical routing, wherein the at least one channel for acoustical routing is connected at one end to the interface and at its other end to the at least one opening, and wherein said transducer fixture is made as a single part produced by additive manufacturing.

## Description

### FIELD

The present disclosure generally relates to a transducer fixture for an ear probe.

The present disclosure further relates to the use of a transducer fixture in an ear probe of an instrument for hearing diagnostics.

The present disclosure further relates to an ear probe for hearing diagnostics comprising a transducer fixture.

The present disclosure further relates to a method for producing a transducer fixture for an ear probe.

### BACKGROUND

In hearing diagnostics, the hearing capacity of the tested individual is assessed using a hearing-diagnostic instrument. The hearing-diagnostic instrument is usually configured to be controlled to emit an audio signal, sometimes combined with providing an air pressure, and acquire a response from the auditory system of the tested individual. Examples of known hearing diagnostics methods include, among others, otoacoustic emissions (OAE), where the sound emitted by the inner ear in response to external auditory stimuli is tested, or tympanometry, where the function of the middle ear and the mobility of the eardrum (tympanic membrane) are tested. Generally, the hearing-diagnostic methods require the insertion of an ear probe into the ear canal of the tested individual.

In general, ear probes containing at least two transducers are used in hearing diagnostics. Current ear probes used in hearing-diagnostic instruments conventionally consist of a number of transducers, at least one speaker and one microphone, delivering acoustic stimulation to the ear and recording acoustic responses from the ear, respectively.

In current ear probes, transducers are typically placed unidirectionally in the ear-probe body to overcome inherent limitations in manufacturing methods such as injection molding. This unidirectional placement may result in a large diameter of the ear probe because the transducers have to be placed side-by-side. This is particularly the case for odd-sized, e.g., circular, transducers. A large-diameter ear probe may be unattractive from design and user-experience points of view. Furthermore, a large-diameter ear probe may be inconvenient for the user and the tested individual because it limits the visibility during ear-probe insertion whilst also interfering with parts of the individual's ear. Furthermore, ear probes with transducers positioned side-by-side may suffer excessively from crosstalk.

To produce conventional ear probes, during assembly, usually a small amount of low viscosity glue is applied to transducers which are then press fitted into a fitting part and left to harden before the entire probe housing is filled with epoxy to keep all the components in place. The epoxy is not allowed to shrink or expand during the hardening process as this will change the location of said transducers. Because the epoxy is used to fill up the entire probe housing thus flowing into all cavities, it is not possible to accelerate the hardening of it with processes such as UV lightning, which results in a prolonged assembly process time.

Therefore, there is a need to provide a solution that addresses at least some of the above-mentioned problems, in particular to provide a transducer fixture which allows for more flexible positioning of the transducer(s). Moreover, against this background, it is an object of the present invention to provide an advantageous use of a transducer fixture and an advantageous ear probe for hearing diagnostics comprising a transducer fixture as well as a method for producing a transducer fixture for an ear probe.

### SUMMARY

According to a first aspect, the above-mentioned object is solved by a transducer fixture for an ear probe. The transducer fixture for an ear probe may comprise an interface for a probe tip. The interface for a probe tip may be configured in a way that a probe tip can be connected to the interface, in particular a probe tip that may be inserted into the tested individual's ear canal for hearing-diagnostic purposes. Preferably, the interface allows for easy installation and removal of the probe tip. Moreover, the interface is preferably compatible with a variety of probe tips which can be fitted to the transducer fixture via the interface. For this purpose, the shape of the interface may be designed in a specific shape so that an accordingly designed probe tip can be plugged onto the interface. It may be provided that the interface is designed as a protruding element on a flat element so that a probe tip can be placed over the protruding element onto the flat element. The protruding element in the sense of a female-male connection can be designed as the negative form of a corresponding form of the probe tip. At the protruding element of the interface, channels for acoustical and/or barometric routing may end so that they can be connected to corresponding channels in the probe tip to be connected at the interface.

The transducer fixture for an ear probe may comprise at least one opening for attaching a transducer.

The transducer fixture for an ear probe may comprise at least one cavity (e.g., dimensioned) for receiving a transducer.

The transducer fixture for an ear probe may comprise at least one passage (e.g., dimensioned) for receiving a transducer.

An opening/cavity/passage for attaching a transducer may be a part or an area of the transducer fixture, in particular a part or an area of the body of the transducer fixture, which is designed to attach a transducer to it, for instance, a part which is hollow and open on at least one side to receive the transducer and attach it to this part. The transducer fixture may be designed to receive and firmly hold in place a transducer. The openings/cavities/passages may be designed so that the transducers can be held into the openings/cavities/passages as a press fit.

The opening/cavity/passage may be configured to at least partially surround a transducer. The opening/cavity/passage may be at least partially dimensioned for a transducer.

The opening/cavity/passage may have a tapered shape towards the channel for acoustical routing.

In other words, the cross sectional area of the opening/cavity/passage may decrease towards the channel for acoustical routing.

The transducer fixture for an ear probe may comprise at least one channel for acoustical routing. Acoustical routing refers to the use of tubes or channels to transfer sound waves e.g., from the ear canal to a transducer (or reverse), for example in an ear probe. Acoustical routing thus ensures that a transducer, for example a microphone, receives sound waves from the correct position within the ear canal to capture accurate sound data. The channel for acoustical routing may be designed so as to guide sound waves from a transducer (attached to the opening) to the probe tip at the interface of the transducer fixture.

For example, a channel for acoustical routing may have the form of a circular hollow tube and may extend from an opening (for attaching a transducer) to the interface of the transducer fixture. The channel may be designed to minimize interference and external noise, ensuring that the transducer (e.g., a microphone) may pick up the sound emitted from the eardrum without contamination from ambient sounds.

The at least one channel for acoustical routing may be connected at one end to the interface and at its other end to the at least one opening. This way the at least one channel for acoustical routing can guide sound waves from the transducer (a loudspeaker) attached to the opening to the interface (i.e., to a probe tip connected at the interface), and can guide sound waves reflected from the tested individual's ear to a transducer (a microphone) to be collected for analysis purposes.

For example, the at least one channel for acoustical routing may have a complex form, for instance the channel may have at least in parts a helical structure or a structure which is at least in sections twisted around the longitudinal axis of the channel. Moreover, the structure of the at least one channel for acoustical routing may have curves and bends.

The transducer fixture may be one unit.

In other words, the transducer fixture may be made as a single part (body or unit) produced by additive manufacturing. This allows for greater design flexibility, reduced waste, and the ability to create complex and intricate structures of the transducer fixture that are challenging or impossible to achieve with traditional methods such as injection molding. The transducer fixture may be made based on a digital design or model. The transducer fixture may be built up layer by layer from this model. The layers can be as fine as a fraction of a millimeter. Further, having the transducer fixture produced in one part by additive manufacturing mitigates the risk compared to a multipart assembly of having airgaps or acoustic leaks. This production method furthermore is time- and cost-effective as waste material can be significantly reduced.

Another advantage of additive manufacturing of the transducer fixture, is the interface for a probe tip. Using additive manufacturing allows altering the exit channel's geometry at the interface which allows for a smaller diameter of the attached probe tip.

That the transducer fixture may be made as a single part produced by additive manufacturing also has a great benefit for the assembly process. The additive-manufactured transducer fixture can be printed in the shape of, for example, a cylinder which can be inserted into an ear-probe housing. The amount of glue (e.g., for transducer attachment) can be reduced significantly as the printed geometry can create cavities that allows easy mounting and uniform thickness of glue that can be UV hardened, thus reducing assembly time.

The specific additive manufacturing method used may be, for example, Fused Deposition Modeling (FDM), Stereolithography (SLA), Digital Light Processing (DLP), Selective Laser Sintering (SLS), or Electron Beam Melting (EBM). For the additive manufacturing process, multiple print heads may be used to enable the use of different materials in a single print. This multi-material printing capability can be used to create composite structures, including lamella patterns.

According to a second aspect, the above-mentioned object is solved by the use of the afore-mentioned transducer fixture or an embodiment thereof in an ear probe of an instrument for hearing diagnostics. This way, the hearing diagnostics can be made more pleasant for the tested individual as the probe tip can be designed smaller thanks to the channel's geometry at the interface due to additive manufacturing of the transducer fixture. Furthermore, the hearing diagnostics may be more accurate, as the design of the at least one channel for acoustical routing may be chosen such that contamination of the diagnostic measurement is at least reduced.

According to a third aspect, the above-mentioned object is solved by an ear probe for hearing diagnostics comprising the afore-mentioned transducer fixture or an embodiment thereof. This way, the advantages described for the above-mentioned transducer fixture, or an embodiment thereof can be used advantageously when conducting hearing diagnostics using an ear probe. Thanks to the smaller possible design of the interface, the probe tip of the ear probe can be smaller in size so that the ear probe is more comfortable for the tested individual. Moreover, the ear probe for hearing diagnostics comprising the afore-mentioned transducer fixture or an embodiment thereof allows for more exact measurements during hearing diagnostics as acoustic leaks are reduced.

The ear probe may additionally contain a probe tip, which may be connected to the transducer fixture via the interface. Moreover, the ear probe may contain a housing, in which the transducer fixture may be inserted/contained. The ear probe may further contain a light source, such as an LED or halogen bulb, to illuminate the ear canal and provide a clear view of the ear canal of the tested individual.

According to a fourth aspect, the above-mentioned object is solved by a method for producing a transducer fixture for an ear probe, especially the afore-mentioned transducer fixture or an embodiment thereof. This allows for creating a transducer fixture with greater design flexibility, reduced waste, and the ability to create complex and intricate structures of the transducer fixture that are challenging or impossible to achieve with traditional methods such as injection molding. Further, having the transducer fixture produced in one part by additive manufacturing mitigates the risk compared to a multipart assembly of having airgaps or acoustic leaks.

The method for producing a transducer fixture for an ear probe, especially the afore-mentioned transducer fixture or an embodiment thereof, may comprise the step of providing a three-dimensional model of a transducer fixture for an ear probe. This way complex three-dimensional structures of the transducer fixture can be simulated by the model and the design can be defined in detail in advance. Moreover, the design of the transducer fixture can easily be amended by amending the model. The three-dimensional model may be or contain a design file specifying the exact geometry and dimensions of the part.

The method for producing a transducer fixture for an ear probe, especially the afore-mentioned transducer fixture or an embodiment thereof, may optionally comprise a preprocessing step, in which the three-dimensional model is prepared for additive manufacturing.

The afore-mentioned transducer fixture or an embodiment thereof, may comprise an interface for a probe tip, at least one opening for attaching a transducer, at least one channel for acoustical routing, wherein the at least one channel for acoustical routing is connected at one end to the interface and at its other end to the at least one opening.

The method for producing a transducer fixture for an ear probe, especially the afore-mentioned transducer fixture or an embodiment thereof, may comprise the step of choosing a material for the transducer fixture. The choice of materials may depend on the desired properties of the transducer fixture and the method for producing the transducer fixture, such as the 3D printing or additive manufacturing technology, to be used.

The method for producing a transducer fixture for an ear probe, especially the afore-mentioned transducer fixture or an embodiment thereof, may comprise the step of additive manufacturing of the transducer fixture according to the three-dimensional model. In particular, the step of additive manufacturing of the transducer fixture may comprise applying material layer by layer to build up the transducer fixture. Additive manufacturing or additive layer manufacturing may be understood as 3D printing, a computer-controlled process that creates three-dimensional objects by depositing materials, usually in layers. This results in enormous flexibility and design freedom, for example in the production of prototypes and also increasingly in series production.

By utilizing additive manufacturing, transducer fixtures are no longer restricted in a way that transducers must be placed unidirectionally or by the limited design freedom resulting from injection molding or various other manufacturing methods. An additive-manufactured transducer fixture, where the channels for acoustical and/or barometric routing from multiple transducers or airflows can be woven or intertwined in a complex pattern, can supply the design freedom to decrease the diameter of an ear probe by placing the transducers differently in the 3D space all in one part. With the design freedom provided by additive manufacturing, various precautions can be taken to avoid crosstalk between at least two transducers, for example between speaker and microphone, such as isolating the transducers in a full-body press fit or creating a pattern of slats between transducers to absorb the vibrations.

The transducer fixture may comprise at least one channel for barometric routing, and the at least one channel for barometric routing may be connected at one end to the interface. For example, the at least one channel may at the other end (i.e., the at least one outlet) be connected to an air-pump arrangement for controlling the pressure in the ear canal. Thereby, the air-pump arrangement may be configured to control the air pressure inside the ear canal, when the ear probe has been inserted into said ear canal. The air-pump arrangement may be connected to (or comprise) a pressure sensor for sensing the pressure in the ear canal in the space between the probe tip and the tympanic membrane.

The air-pump arrangement may comprise at least one pump, such as a membrane, piston, peristaltic, or gear pump. For example, the air-pump arrangement may comprise two pumps operated in combination.

The transducer fixture may comprise at least two openings each for attaching a transducer and at least two channels for acoustical routing, and each of the at least two channels for acoustical routing may be connected at one end to the interface and at its other end to one of the at least two openings, so that the openings are connected to the interface by one channel each. One of the transducers may be a microphone and the other one of the transducers may be a speaker. The microphone may convert variations of sound pressure, thus, sound waves, into variations of electrical potential, and the speaker may perform the reverse function.

The transducer fixture may comprise at least three openings for attaching a transducer and at least three channels for acoustical routing, and each of the at least three channels for acoustical routing may be connected at one end to the interface and at its other end to one of the at least three openings, so that the openings are connected to the interface by one channel each. In this way, the transducer fixture may be used in an ear probe used for specialized hearing diagnostics tests that require multiple transducers and measurements and specific capabilities.

The interface of the transducer fixture may have a number of apertures corresponding to the number of channels, and each channel may open into one of the apertures. The apertures may be used to connect at the interface a (reusable) probe tip with corresponding apertures so that the channels for acoustical and/or barometric routing are continued within the probe tip via the apertures. The apertures of the interface of the transducer fixture may have a circular shape in cross section. The size of the cross section, especially of the diameter of the circular cross section, may vary among the apertures.

The transducer fixture may have a pattern of slats at least in one area. This helps to absorb vibrations between the openings of the transducer fixture and hence between transducers attached to in the openings. Hence an improved sound isolation and diminution of sound contamination and crosstalk can be achieved. A pattern of slats can be understood in a way that each of the openings for attaching a transducer may be at least partially surrounded by one or more cavities for absorbing vibrations. The transducer fixture may generally have a pattern of slats or a lamella structure. Alternatively, the transducer fixture may comprise a pattern of slats or a lamella structure that only extend in some areas of the transducer fixture, for example in areas between/surrounding the different openings. A lamella structure may consist of thin, parallel layers of sheets. Typically, the design of the lamella structure or the pattern of slats may be created in a 3D modeling software (e.g., using CAD tools) for producing the transducer fixture as a single part by additive manufacturing.

The transducer fixture may be at least partially designed as a solid body. This way, the rigidity and therefore the durability of the transducer fixture is increased. In particular, substantially the whole transducer fixture may be designed as a solid body. An at least partial design as a solid body helps to isolate the openings and hence the transducers attached to the openings from each other and helps to avoid creation of artefacts by uncontrolled voids in the transducer fixture

The transducer fixture may be substantially in the form of a cylinder. This way a rather easy to produce and easy to handle form of a transducer fixture and thus preferentially of an ear probe, for which the transducer fixture is used, may be achieved. For example, a rather simple geometry for the enclosure of the transducer fixture, such as a cylindrical enclosing, may be chosen. An essentially cylindrical shape is understood to be a shape that can essentially be described by a cylinder, but which may nevertheless have slight deviations from a cylindrical shape.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effects will be apparent from and elucidated with reference to the illustrations described hereinafter in which:
- Fig. 1: schematically illustrates a section view of an example of an ear probe according to the second aspect with a transducer fixture according to the first aspect comprising three openings for attaching a transducer and three channels for acoustical routing;
- Fig. 2: illustrates a further example of a transducer fixture for an ear probe according to the first aspect comprising three openings for attaching a transducer and three channels for acoustical routing in a schematic perspective view from diagonally below;
- Fig. 3: illustrates a front view of the interface of the example of a transducer fixture of Fig. 2;
- Fig. 4a-b: illustrates sectional views of the example of a transducer fixture of Figs. 2 and 3; and
- Fig. 5: illustrates a sectional view of the example of a transducer fixture of Figs. 2 to 4 with superimposed internal structures showing the channels for acoustical and barometric routing.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

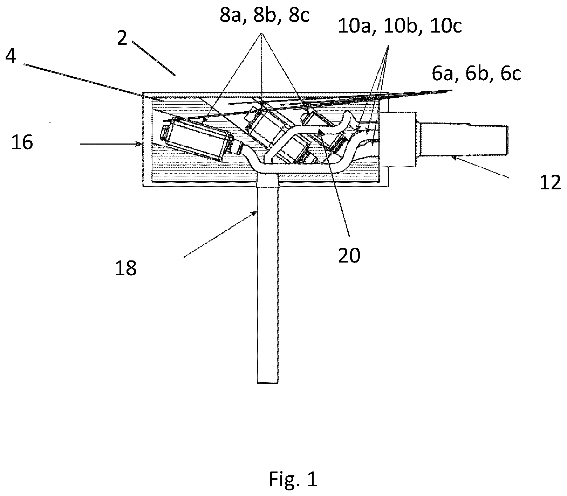

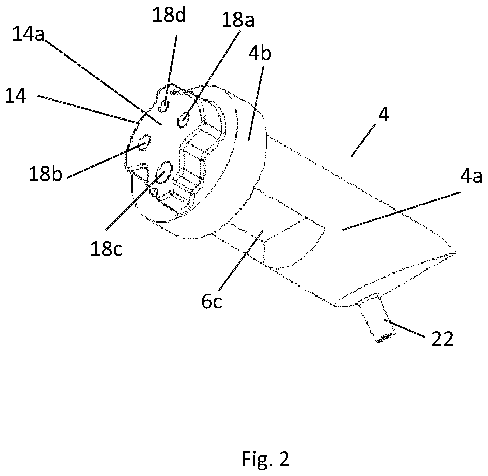

Fig. 1 schematically illustrates a section view of an ear probe 2 according to the second aspect with a transducer fixture 4 according to the first aspect comprising at least three openings 6a, 6b, 6c for attaching a transducer 8a, 8b, 8c and three channels for acoustical routing 10a, 10b, 10c. The transducers 8a, 8b, 8c may for example comprise one or more speakers and a microphone. The ear probe may further comprise a (deposable) probe tip 12 which may be fixed to the ear probe via an interface, such as the interface 14 shown in Fig. 2. The channels for acoustical routing 10a, 10b, 10c are each connected at one end to the interface 14 and at its other end to one of the three openings 6a, 6b, 6c. Within the ear probe 2, the transducer fixture 4 is contained within a probe enclosure 16 (i.e., a housing of the ear probe 2). In the shown example, the transducer fixture 4 and the probe enclosure 16 are each substantially in the form of a cylinder. At the bottom of the probe enclosure 16, a tubing 18 for connecting an air-pump arrangement (not shown) to a channel for barometric routing 20.

The channel for barometric routing 20 extends from said tubing 18 through the body of the transducer fixture 4 to said interface 14. The transducer fixture 4 may have a pattern of slats. The transducer fixture 4 is made as a single part produced by additive manufacturing, which enables the design of the complex and interwoven channels for acoustical and barometric routing 10a, 10b, 10c, 20.

Fig. 2 illustrates a further example of a transducer fixture 4 for an ear probe 2 according to the first aspect comprising three openings for attaching a transducer (not shown) and three channels for acoustical routing (not shown) in a schematic perspective view from diagonally below. The transducer fixture 4 in this example is shown without a probe enclosure so that the geometry of the transducer fixture 4 is better visible. Here, the transducer fixture 4 comprises a main body 4a in the form of a solid body. The main body 4a has essentially the form of a cylinder, whose height is greater than its diameter. At one end of the main body 4a, an adapter piece 4b connects to the main body 4a. The adapter piece 4b has the form of a cylinder, whose height may be smaller than its diameter. The interface 14 may connect to the adapter piece 4b at an opposite side than the main body 4a. The interface 14 may be designed in a way to be able to receive a probe tip of an ear probe in which the transducer fixture 4 may be used.

Here, the interface 14 is formed as a protruding element of a complex geometric shape with many partly rounded edges and a planar surface 14a at the end of the interface 14 opposite the end in connection with the adapter piece 4b of the transducer fixture 4. The interface 14 may be formed as the negative element of a probe tip with an interface forming the corresponding positive element so that the probe tip can easily be connected to the transducer fixture and hence to the ear probe the transducer fixture 4 is used for.

The interface 14 may have a number of apertures 18a, 18b, 18c, 18d corresponding to the number of channels for acoustical and barometric routing 10a, 10b, 10c, 20, wherein each channel 10a, 10b, 10c, 20 opens into an aperture 18a, 18b, 18c, 18d. The apertures 18a, 18b, 18c, 18d can be connected to corresponding apertures of a probe tip so that the channels serve as guiding channels for the transmittal of sound signals between the ear of the individual to be tested and the transducers 8a, 8b, 8c attached in the openings 6a, 6b, 6c of the transducer fixture 4 as well as for control of air-pressure. Further, in Fig. 2, an outlet 22 is visible. The channel for barometric routing 20 may be connected at its other end to the outlet 22. The outlet 22 may be connected to a tubing (not shown) for connecting an air-pump arrangement (not shown) to the channel for barometric routing 20.

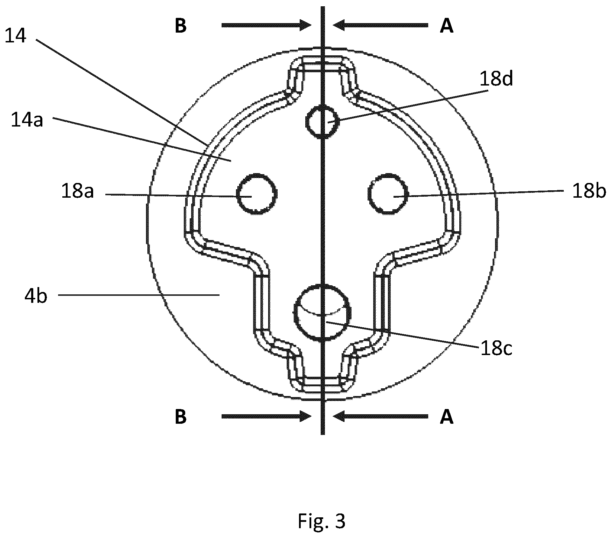

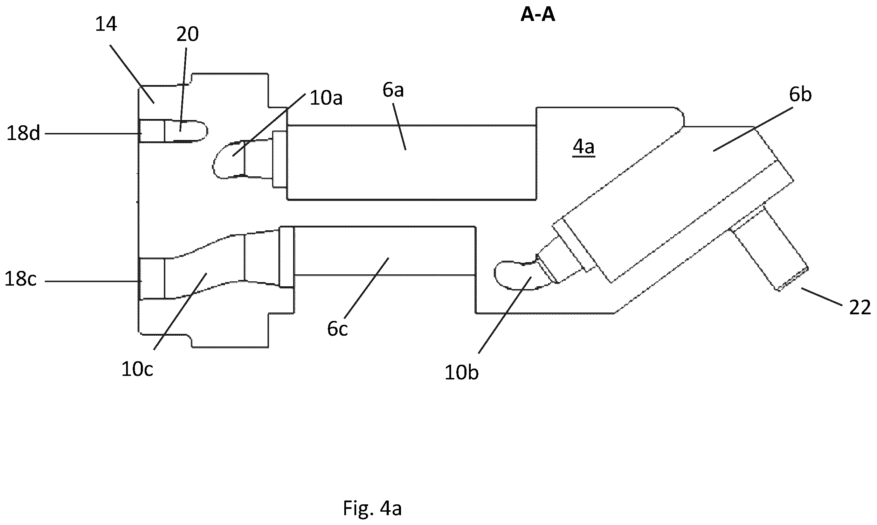

In Fig. 3, a front view of the interface of the example of a transducer fixture 4 of Fig. 2 is shown. The adapter piece 4b connecting the interface 14 to the main body 4a can be seen in this front view. The main body 4a lies behind the adapter piece 4b in this view and thus cannot be seen in this figure. The apertures 18a, 18b, 18c, 18d, whose number corresponds to the number of channels for acoustical and barometric routing 10a, 10b, 10c, 20, are visible. The apertures 18a, 18b, 18c, 18d are arranged at a distance from each other and distributed across the planar surface 14a of the interface 14. The apertures 18a, 18b, 18c, 18d in this example all have a circular cross-section, however the diameter of the cross-sections of the apertures 18a, 18b, 18c, 18d differ from each other in this example. Here, the diameter of the cross-section of the aperture 18c, which is positioned at the bottom of the planar surface 14a of the interface 14, has the biggest diameter. The diameter of the cross-section of the aperture 18d, which is positioned at the top of the planar surface 14a of the interface 14, has the smallest diameter. The diameters of the cross-sections of the apertures 18a, 18b, which are positioned on the remaining two sides of the planar surface 14a opposite each other, are approximately equal in size and smaller than the diameter of the aperture 18c as well as bigger than the diameter of the aperture 18d. In Fig. 3, the letters A and B designate cutting axes A-A and B-B of the sectional views shown in Fig. 4a-b.

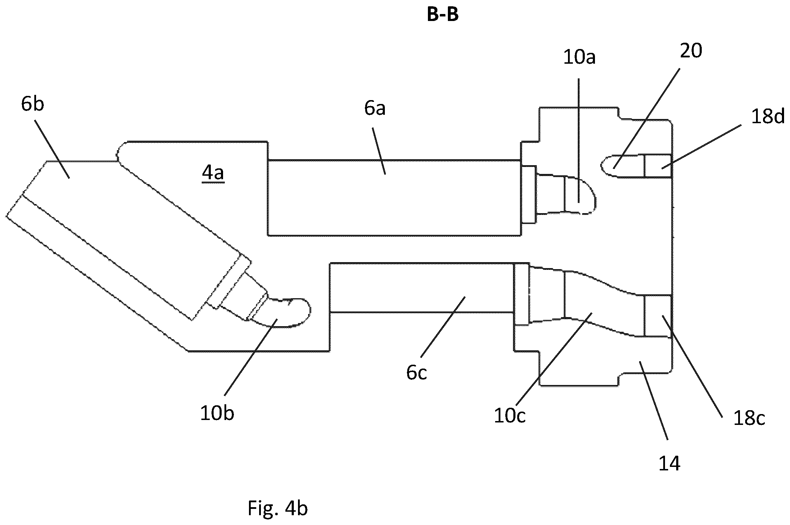

Fig. 4a-b illustrate sectional views of the example of a transducer fixture 4 of Figs. 2 and 3, namely Fig. 4a illustrates a sectional view along the cutting axis labeled A-A in Fig. 3, and Fig. 4b illustrates a sectional view along the cutting axis labeled B-B.

In Fig. 4a and 4b, the openings 6a, 6b, 6c, each for attaching a transducer, can be seen. Furthermore, parts of the channels for acoustical routing 10a, 10b, 10c can be seen. Each of the channels for acoustical routing 10a, 10b, 10c is connected at one end to the interface 14 and at its other end to one of the openings 6a, 6b, 6c, so that the openings 6a, 6b, 6c are connected to the interface 14 by one channel 10a, 10b, 10c each. Furthermore, parts of the channel for barometric routing 20 can be seen. The channel for barometric routing 20 is connected at one end to the interface 14. The transducer fixture 4 comprises an outlet 22 at its main body 4a (see also Fig. 2) and the channel for barometric routing 20 is connected at its other end to the outlet 22.

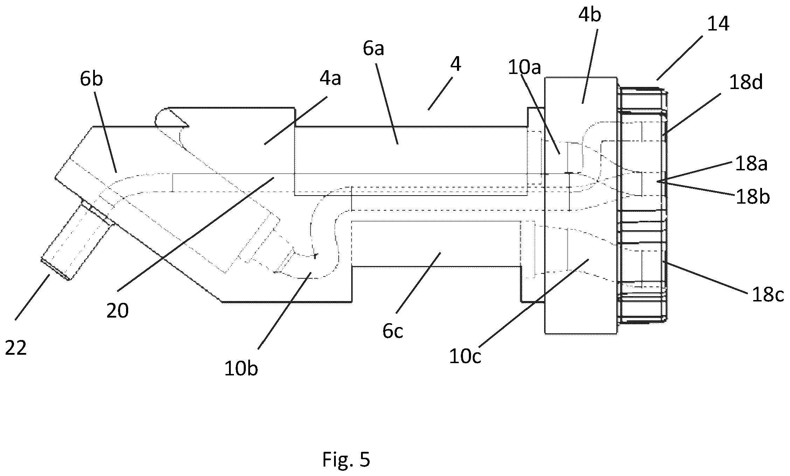

Fig. 5 illustrates a sectional view of the example of a transducer fixture 4 of Figs. 2 to 4 with superimposed internal structures showing the channels for acoustical and barometric routing 10a, 10b, 10c, 20. Additional to the features visible in Fig. 4a-b, the openings 6a, 6b, 6c and the apertures 18a, 18b, 18c, 18d, the internal structure of the channels for acoustical and barometric routing 10a, 10b, 10c, 20 can be seen in Fig. 5. As can be seen, the internal structure of the channels 10a, 10b, 10c, 20 inside the main body 4a and adapter piece 4b is complex and the openings 6a, 6b, 6c as well as the channels for acoustical and barometric routing 10a, 10b, 10c, 20 can be positioned not unidirectionally relative to each other. Thanks to making the transducer fixture 4 as a single part produced by additive manufacturing, an interwoven structure of the channels for acoustical and barometric routing 10a, 10b, 10c, 20 is possible thus permitting an increased design flexibility and taking various precautions to avoid crosstalk between transducers 8a, 8b, 8c attached to the openings 6a, 6b, 6c. Hence an improved ear probe to be used in hearing diagnostics is achieved.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects. Reference to an element in the singular is not intended to mean "one and only one" unless specifically so stated, but rather "one or more." Unless specifically stated otherwise, the term "some" refers to one or more.

Accordingly, the scope should be judged in terms of the claims that follow.

## Claims

1. Transducer fixture for an ear probe, comprising:
- an interface for a probe tip,
- at least one cavity dimensioned for receiving and attaching a transducer,
- at least one channel for acoustical routing,
- wherein the at least one channel for acoustical routing is connected at one end to the interface and at its other end to the at least one cavity, and
- wherein said transducer fixture is one unit.

2. Transducer fixture according to claim 1,
- wherein the transducer fixture further comprises at least one channel for barometric routing, and
- wherein the at least one channel for barometric routing is connected at one end to the interface.

3. Transducer fixture according to claim 2,
- wherein the transducer fixture further comprises at least one outlet, and
- wherein the at least one channel for barometric routing is connected at its other end to the at least one outlet.

4. Transducer fixture according to one of claims 1 to 3,
- wherein the transducer fixture comprises at least two cavities each for attaching a transducer and at least two channels for acoustical routing, and
- wherein each of the at least two channels for acoustical routing is connected at one end to the interface and at its other end to one of the at least two cavities, so that the cavities are connected to the interface by one channel each.

5. Transducer fixture according to one of claims 1 to 4,
- wherein the transducer fixture comprises at least three cavities for attaching a transducer and at least three channels for acoustical routing, and
- wherein each of the at least three channels for acoustical routing is connected at one end to the interface and at its other end to one of the at least three cavities, so that the cavities are connected to the interface by one channel each.

6. Transducer fixture according to one of claims 1 to 5,
- wherein the interface has a number of apertures corresponding to the number of channels, and
- wherein each channel opens into an orifice.

7. Transducer fixture according to one of claims 1 to 6,
- wherein the transducer fixture has a pattern of slats or a lamella structure at least partially surrounding the at least one cavity for attaching a transducer.

8. Transducer fixture according to one of claims 1 to 7,
- wherein the transducer fixture is at least partially designed as a solid body.

9. Transducer fixture according to one of claims 1 to 8,
- wherein the transducer fixture is substantially in the form of a cylinder.

10. Use of a transducer fixture according to one of claims 1 to 9 in an ear probe of an instrument for hearing diagnostics.

11. Ear probe for hearing diagnostics comprising a transducer fixture according to one of claims 1 to 9.

12. Method for producing a transducer fixture for an ear probe, especially a transducer fixture according to one of claims 1 to 10, comprising the steps of:
- providing a three-dimensional model of a transducer fixture for an ear probe, especially a transducer fixture according to one of claims 1 to 9, wherein the transducer fixture comprises:
- an interface for a probe tip,
- at least one cavity for attaching a transducer,
- at least one channel for acoustical routing,
- wherein the at least one channel for acoustical routing is connected at one end to the interface and at its other end to the at least one cavity,
- choosing a material for the transducer fixture, and
- additive manufacturing of the transducer fixture according to the three-dimensional model.
